# EUROPEAN PATENT APPLICATION

(11) **EP 3 456 292 A1**
(43) Date of publication of application: **20.03.2019**
(21) Application number: 17796227.1
(22) Date of filing: 11.05.2017
(51) Int. Cl.: A61F 2/02, A61F 2/04

(54) **TISSUE BODY FORMATION DEVICE**

(30) Priority: 12.05.2016 JP 2016096469
(71) Applicant: Shinkan Kogyo K.K., Osaka-shi, Osaka 555-0011 (JP); National Cerebral and Cardiovascular Center, Suita, Osaka 565-8565 (JP)
(72) Inventor: NAKAYAMA, Yasuhide, Suita-shi Osaka 565-8565 (JP); MORIWAKI, Takeshi, Suita-shi Osaka 565-8565 (JP); OIE, Tomonori, Osaka-shi Osaka 555-0011 (JP)
(74) Representative: Westphal, Mussgnug & Partner Patentanwälte mbB
(86) International application number: PCT/JP2017/017923
(87) International publication number: WO 2017/195869

(57) **Abstract**

A tissue body formation device 10 for forming a connective tissue body in an environment where a living tissue material is present is provided with: an inner member 20 having a tissue body formation surface that serves as a surface for forming a connective tissue body; and a covering member which is provided with a covering surface that constitutes an external surface of the tissue body formation device, and that covers a part of the tissue body formation surface. The covering member further has a plurality of connection parts that connect the outer side of the tissue body formation device 10 to the tissue body formation surface. Each of the connection parts has an opening in the covering surface. Each of the openings has a minimum dimension of at least 0.5 mm in a direction along the covering surface. The openings account for 20-40% per unit area of the covering surface.

## Description

### TECHNICAL FIELD

The present invention relates to a tissue body formation device for forming connective tissue.

### BACKGROUND ART

The human body has a self-defense function in which a capsule mainly formed by fibroblast and collagen encapsulates foreign matter. One type of regenerative medicine, which regenerates lost tissues or organs with artifacts, embeds or implants a tissue body formation device as foreign matter in a living body to generate living-body-derived connective tissue from living cells utilizing the self-defense function (see, for example, patent document 1 to 3). The tissue body formation device used as the foreign matter includes two tissue forming surfaces opposed to each other. Living tissue material enters the space between the two tissue forming surfaces to form connective tissues (see, for example, patent document 4).

### PRIOR ART DOCUMENTS

### PATENT DOCUMENTS

Patent Document 1: Japanese Laid-Open Patent Publication No. 2007-312821
Patent Document 2: Japanese Laid-Open Patent Publication No. 2008-237896
Patent Document 3: Japanese Laid-Open Patent Publication No. 2010-094476
Patent Document 4: Japanese Laid-Open Patent Publication No. 2014-030598

### SUMMARY OF THE INVENTION

In actual use of regenerative medicine using a tissue body formation device, there is a need to the burden applied to a living body, in other words, it is strongly required that the a tissue body formation device be implanted within a shorter period of time in an environment where living tissue material exists. In medical treatment using connective tissue formed by a tissue body formation device, there is a continuously demand for increasing the structural precision of the connective tissue. However, when shortening the period of time for implanting the tissue body formation device in the living tissue material, the connective tissue will be locally reduced in size or locally reduced in thickness. Accordingly, although one of shortening the period of time for implanting the tissue body formation device and improving the structural precision of the connective tissue can be achieved but the other cannot. Thus, these two demands have a trade-off relationship.

It is an object of the present invention to provide a tissue body formation device that increases the structural precision of connective tissue without prolonging the implantation time of the tissue body formation device in an environment where living tissue material is present.

One aspect of the present invention that achieves the above object is a tissue body formation device for forming connective tissue in an environment where living tissue material is present. The device includes a covering member having a covering surface that is a surface defining an outer surface of the tissue body formation device, wherein the covering surface covers part of a tissue forming surface that is a surface for forming the connective tissue. The covering member includes a plurality of communication parts through which an outer side of the tissue body formation device is in communication with the tissue forming surface. The communication parts each have an opening in the covering surface. The openings each have a minimal dimension of 0.5 mm or greater in a direction extending along the covering surface. The openings have an occupancy rate of 20% or greater and 40% or less per unit area of the covering surface.

With the tissue body formation device described above, each of the openings has a minimal dimension along the covering surface of 0.5 mm or greater, which prevents clogging of the opening by living tissue material entering the opening before the tissue forming surface is fully covered by the living tissue material. In addition, the occupancy rate of the openings per unit area of the covering surface is 40% or less, which prevents formation of recesses in the connective tissue at locations opposing the openings. Furthermore, the occupancy rate of the openings per unit area of the covering surface is 20% or greater, which makes it possible to obtain the necessary amount of living tissue material reaching the tissue forming surface as the living tissue material moves through the openings. Consequently, it is possible to prevent a prolonged implantation period of the device in an environment where living tissue material is present by obtaining the necessary amount of living tissue material reaching the tissue forming surface, and it is possible to improve the structural precision of the connective tissue by limiting the formation of recesses at locations opposing the openings.

The connective tissue refers to tissue which is composed mainly of collagen and is formed within a living body. For this point, the technology of the present disclosure also includes tissue that corresponds to connective tissue formed within a living body and is formed in an environment outside the living body. In addition, the living tissue material refers to substance needed in forming tissue derived from a living body and includes, for example, animal cells such as fibroblast cells, smooth muscle cells, ES cells and iPS cells, a variety of proteins such as collagen and elastin, sugars such as hyaluronic acid, a cell growth factor that promotes the growth and differentiation of cells, and various physiologically active substances such as cytokine present in the living body. Furthermore, the living tissue material includes material derived from mammals such as a human, a dog, a cow, a pig, a goat, and a sheep, and birds, fish, and other animals, as well as equivalent artificial material. The living tissue material is present in an environment of a living body that includes, for example, mammals such as a human, a dog, a cow, a pig, a goat, and a sheep, and birds, fish, and other animals, and that includes extremity, shoulder, back, abdomen, and the like subcutaneously, and abdominal cavity. Moreover, the living tissue material is present in an environment that is, for example, an artificial environment containing living tissue material.

One aspect of the present invention is a tissue body formation device for forming connective tissue in an environment where living tissue material is present. The device includes an inner member and a covering member. The inner member has a tissue forming surface that is a surface for forming the connective tissue. The covering member configured to be separable from the inner member and including a covering surface that is a surface forming an outer surface of the tissue body formation device and covers part of the tissue forming surface of the inner member. A hollow gap extends between the tissue forming surface and the covering surface. The covering member allows for removal of the connective tissue by cutting connective tissue with which the gap is filled with from other connective tissues formed on the tissue forming surface and separating the inner member and the covering member. The covering member has a plurality of communication parts through which an outer side of the covering member is in communication with the tissue forming surface. Each of the communication parts includes an opening in the covering surface. The openings each have a minimal dimension of 0.5 mm or greater in a direction extending along the covering surface. The openings have an occupancy rate of 20% or greater and 40% or less per unit area of the covering surface. The per unit area is a structural minimum repeating unit of the covering surface.

With the tissue body formation device, connective tissue is obtained by separating the connective tissue in the hollow gap between the tissue forming surface of the inner member and the covering surface of the covering member from the tissue forming surface. In addition, the implantation period of the tissue body formation device in an environment where living tissue material is present is reduced down to a period which is required for the hollow gap between the tissue forming surface and the covering surface to be filled with the connective tissue. Accordingly, this makes it possible to prevent prolongation of an implantation period of the tissue body formation device in the environment where living tissue material is present and to provide connective tissue with improved structural precision.

In the tissue body formation device, a distance between the tissue forming surface and the covering member may be 0.5 mm or greater. With the tissue body formation device, the distance between the tissue forming surface and the covering member is 0.5 mm or greater, which prevents clogging of a gap between the tissue forming surface and the covering member caused by living tissue material entering the gap before the tissue forming surface is fully covered by the living tissue material. Consequently, this can further enhance the structural precision of the connective tissue.

In the tissue body formation device, a distance between the tissue forming surface and the covering member may be 5.0 mm or less. With the tissue body formation device, the distance between the tissue forming surface and the covering member is 5.0 mm or less, which prevents the formation of parts in the gap between the tissue forming surface and the covering member that is not filled with connective tissue. This further enhances structural precision of the connective tissue.

In the tissue body formation device, the communication part may have a depth of 2.0 mm or less. With the tissue body formation device, the communication part has a depth of 2.0 mm or less, which prevents clogging of the communication part before the tissue forming surface is fully surrounded by living tissue material entering into the communication part. Thus, this can further enhance the structural precision of the connective tissue.

In the tissue body formation device, the distance between adjacent ones of the openings may be 2.0 mm or greater and 5.0 mm or less. With the tissue body formation device, the distance between the openings is 2.0 mm or greater, which allows each of the openings to function separated in relation with the living tissue material. In addition, since the distance between adjacent openings is 5.0 mm or less, the occurrence of differences in the amount of living tissue material entering the openings is prevented or restricted. This can result in further improved structural precision of connective tissue.

In the tissue body formation device, the covering member may include a curved tubular part, and the covering surface may include an outer surface of the tubular part. Further, the covering member may include a tubular portion shaped to have multiple rings, and the covering surface may include an outer surface of the tubular portion. According to these tissue body formation devices, it is possible to effectively provide a covering member with a prolonged dimension in the extending direction within a limited volume where the tissue body formation device is implanted.

In the tissue body formation device, the tissue forming surface may include a surface of an annular portion shaped to have multiple rings in conformance with the tubular portion and is located inside the tubular portion. With the tissue body formation device, it becomes possible to form tubular connective tissue extending in the extending direction of the covering member. It is also possible to provide connective tissue with effectively prolonged dimension in the extending direction within a limited volume where the tissue body formation device is implanted.

In the tissue body formation device, the tubular portion may include, at an end portion of the tubular portion in the extending direction, a supporting part for supporting the tissue forming surface so that a center of the annular portion as viewed from the extending direction coincides with a center of the tubular portion as viewed from the extending direction. With the tissue body formation device, it is possible to prevent or restrict differences in the thickness of the tubular connective tissue because the tissue forming surface is supported inside the tubular portion so that the center of the annular portion as viewed from the extending direction coincides with the center of the tubular portion as viewed from the extending direction.

In the tissue body formation device, the covering member may be shaped to include multiple rings so that a large diameter annular element and a small diameter annular element are spaced part in the radial direction by a distance of 0.5 mm or greater. With the tissue body formation device, the distance between adjacent annular elements is 0.5 mm or greater to reduce non-entry of living tissue material into a gap between adjacent annular elements. Thus, it becomes possible to provide connective tissue with improved structural precision in the extending direction.

In the tissue body formation device, the communication part may be a first communication part, and the covering member may including a tubular shape extending one direction and have, at an end surface of the covering member in the extending direction, a second communication part through which an outer side of the covering member is in communication with an inner side of the covering member. With the tissue body formation device, it is possible to improve structural precision of the connective tissue including the end portion of the connective tissue because living tissue material also enters from the outside to the inside of the covering member through the second communication part at the end surface of the covering member in the extending direction.

### EFFECT OF THE INVENTION

The tissue body formation device according to the present invention allows the structural precision of connective tissue to be increased without prolonging the implantation time of the tissue body formation device in an environment where living tissue material is present.

### BRIEF DESCRIPTION OF DRAWINGS

Fig. 1 is a perspective view illustrating a perspective structure of a tissue body formation device according to a first embodiment.
Fig. 2 is a net of an outer member of the first embodiment in which an outer surface of the outer member is cut and opened and illustrates the relationship between the surface area of the outermost surface of the outer member and areas of its openings.
Fig. 3 is a perspective view illustrating a process for forming connective tissue using the tissue body formation device of the first embodiment and shows procedures (a) to (f) in order by which the tissue body formation device is implanted inside a living body.
Fig. 4 is a diagram illustrating an example of connective tissue formed by the tissue body formation device of the first embodiment.
Fig. 5A is a partial cross-sectional view of the tissue body formation device of the first embodiment illustrating part of a front cross-sectional structure of the device, and Fig. 5B is a partial cross-sectional view of the tissue body formation device illustrating part of a side cross-sectional structure of the device of the first embodiment.
Fig. 6A is a partial cross-sectional view of a tissue body formation device of a comparative example illustrating part of a front cross-sectional structure of the device, and Fig. 6B is a partial cross-sectional view of the tissue body formation device of the comparative example, illustrating part of a side cross-sectional structure of the device.
Fig. 7A is a partial cross-sectional view of a tissue body formation device of a comparative example illustrating part of a front cross-sectional structure of the device, and Fig. 7B is a partial cross-sectional view of the tissue body formation device of the comparative example illustrating part of a side cross-sectional structure of the device.
Fig. 8 is a diagram illustrating the test result of each test example conducted on the tissue body formation device of the first embodiment.
Fig. 9 is a diagram illustrating a test result of each test example conducted on the tissue body formation device of the first embodiment.
Fig. 10 is a perspective view of a tissue body formation device of a second embodiment illustrating its perspective structure.
Fig. 11 is a perspective view of an upper outer member of the second embodiment illustrating a perspective structure thereof.
Fig. 12 is a perspective view of a lower outer member of the second embodiment illustrating its perspective structure.
Fig. 13 is a perspective view of an inner ring-shaped member of the second embodiment illustrating its perspective structure.
Fig. 14 is a perspective view of a modified tissue body formation device illustrating its perspective structure.
Fig. 15 is a perspective view of another modified tissue body formation device illustrating its perspective structure.

### DESCRIPTION OF EMBODIMENTS

### FIRST EMBODIMENT

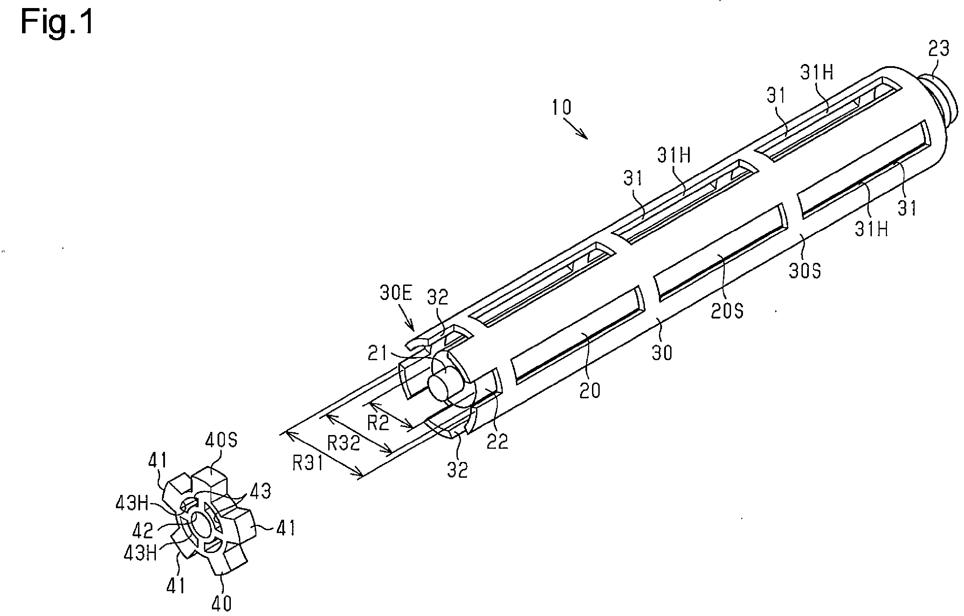

A tissue body formation device according to a first embodiment will be described with reference to Figs. 1 to 7.

As illustrated in Fig. 1, the tissue body formation device 10 is embedded or implanted in an environment where living body material is present to form connective tissue. The tissue body formation device 10 includes an inner member 20, an outer member 30 as an example of a covering member, and a lid 40.

The inner member 20 has a bar-like shape extending in one direction. The inner member 20 includes, for example, a shaft member 21, made of acrylic resin and extending in an extending direction of the inner member 20, and an inner tubular member 22, made of silicone resin. The shaft member 21 has a length that is greater than that of the inner tubular member 22 in an extending direction of the shaft member 21. The shaft member 21 is surrounded by the inner tubular member 22 so that one end of the shaft member 21 in the extending direction of the shaft member 21 protrudes from a corresponding one of the ends of the inner tubular member 22.

The other end of the shaft member 21 in the extending direction is an attached portion 23 that protrudes from a corresponding end of the outer member 30 and is used when a tissue body formation device 10 is removed from an environment where living tissue material is present. The inner tubular member 22 has an outer surface that defines an inside surface 20S representing an example of a tissue forming surface for forming connective tissue. The inner member 20 has an outer diameter that corresponds to an outer dimeter of the inner tubular member 22, which defines a gap inner diameter R2.

The outer member 30 extends in an extending direction of the inner member 20 and has a tubular shape having a size in which the inner member 20 can be inserted. The outer member 30 is made of acrylic resin, for example, and extends in the extending direction of the inner member 20. The exterior surface of the outer member 30 has an outermost surface 30S that represents an example of a covering surface defining the exterior surface of the tissue body formation device 10 and partially surrounding the inside surface 20S. The inside surface 20S functions to form connective tissue. The outer member 30 includes a plurality of first communication parts 31 through which an outer side of the outer member 30 is in communication with the inside surface 20S. Each of the first communication parts 31 has a first opening 31H at the outermost surface 30S. An end portion 30E of the outer member 30 in an extending direction of the outer member 30 is provided with multiple fitted portions 32 extending from an end surface of the outer member 30 in the extending direction at regular intervals that are arranged in a circumference direction of the outer member 30. The end portion 30E of the outer member 30 in the extending direction is closed by the lid 40.

A hollow gap, for example, a tubular gap, is formed between the inside surface 20S of the inner member 20 and the outer member 30. As illustrated in Fig. 1, the outer member 30 has an outer diameter that is an outermost diameter R31 and an inner diameter that is a gap outer diameter R32. The gap outer diameter R32 is greater than the gap inner diameter R2. One half of the difference between the gap outer diameter R32 and the gap inner diameter R2 (= (gap outer diameter R32 - gap inner diameter R2) / 2) defines a thickness of a gap in which connective tissue is formed and which defines a thickness of the connective tissue filled in the gap. The gap has in its radial direction a width (= (gap outer diameter R32 - gap inner diameter R2) / 2) that is preferably between 0.5 mm and 5.0 mm. When the gap has a width of 0.5 mm or greater, living tissue material entering the gap during expansion does not remain in the gap and thus does not clog the passage in which the living tissue material expands. In addition, when the width of the gap is 5.0 mm or greater, this prevents formation of a portion in the gap that will not be filled with connective tissue. One half of the difference between the outermost diameter R31 and the gap outer diameter R32 (= (outermost diameter R31 - gap outer diameter R32) / 2) defines a thickness of the outer member 30 in the radial direction and also defines a depth of first communication parts 31. The depth of the first communication parts 31 is preferably 2.0 mm or less. It is preferred that the thickness be as less as possible. When the depth of the first communication parts 31 is 2.0 mm or less, living tissue material entering the first openings 31H are easily prevented from remaining in the first communication parts 31.

The lid 40 has a disk-like shape having the same diameter as the outer member 30. The lid 40 has a circumference surface 40S that protrudes outward in a radial direction of the lid 40. Multiple fitting claws 41 are arranged in a circumference direction of the lid 40 at regular intervals. The fitting claws 41 are configured so as to fit or engage with the fitted portions 32. Each of the fitting claws 41 is fitted with a corresponding fitted portion 32 to attach the lid 40 to the end portion 30E of the outer member 30 in the extending direction.

The lid 40 has a support opening 42 that extends through the center of the lid 40 in the extending direction of the outer member 30. When the lid 40 is attached to the outer member 30, the shaft member 21 of the inner member 20 is fitted into the support opening 42. In addition, the lid 40 includes around the support opening 42 a plurality of second communication parts 43 that extend through the lid 40 in the extending direction of the outer member 30. Each of the second communication parts 43 has at an end surface of the lid 40 an arcuate second opening 43H. Each second opening 43H has an opening width that is preferably 0.5 mm or greater. When the opening width of the second opening 43H is 0.5 mm or greater, the connective tissue formed near the lid 40 easily obtains the necessary thickness. This prevents living tissue material entering the second opening 43H from remaining inside the second communication part 43 before it reaches the gap between the inner member 20 and the outer member 30. Consequently, living tissue material does not clog the second communication part 43. The lid 40 has a thickness that corresponds to a depth of the second communication part 43, which is preferably 2.0 mm or less. Preferably, the thickness is as less as possible. When the depth of the second communication part 43 is 2.0 mm or less, living tissue material entering the second opening 43H is easily prevented from remaining inside the second communication part 43.

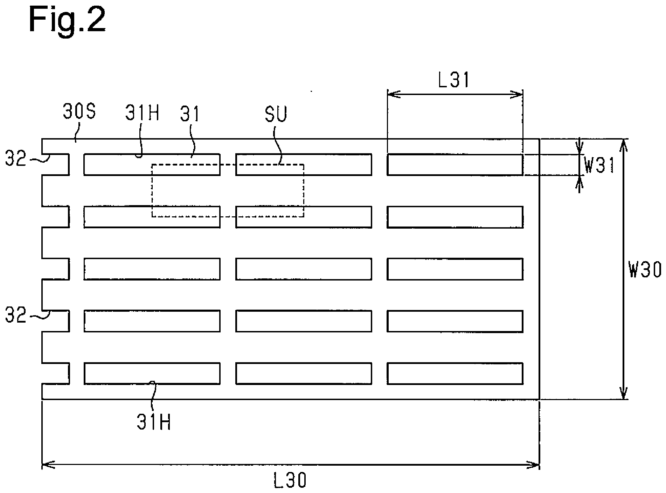

Next, the occupancy rate of the first openings 31H per unit area of the outermost surface 30S will be described. Fig. 2 is a net in which the outermost surface 30S of the outer member 30 is cut and spread out in the extending direction thereby exemplifying an outermost surface 30S provided with first openings 31H in an arrangement of five rows by three columns.

As illustrated in Fig. 2, the outermost surface 30S of the outer member 30 has a length L30 in the extending direction of the outer member 30 and a width W30 in the circumference direction of the outer member 30. The outermost surface 30S of the outer member 30 has an area that is the length L30 times the width W30. Each of the first openings 31H has an opening length L31 in the extending direction of the outer member 30 and an opening width W31 in the circumference direction of the outer member 30. The opening width W31 is the minimum dimension that the first opening 31H has in the direction along the outermost surface 30S. Each first opening 31H has an area that is represented by the opening length L31 times the opening width W31. The first openings 31H of 5 rows times 3 columns are arranged at regular intervals in the outermost surface 30S of the outer member 30 in the extending and circumference directions. The outermost surface 30S of the outer member 30 is provided with unit regions SU each of which defines a structural minimal repeating unit on the outermost surface 30S. Each of the unit regions SU includes parts of two first openings 31H adjacent to each other in the extending direction and parts of two first openings 31H adjacent to each other in the circumference direction.

In the outermost surface 30S of the outer member 30, an area that the unit region SU has defines a unit area. In the unit area, the ratio that the first openings 31H occupy is 20% or greater and 40% or less. In other words, a sum of the areas of all the first openings 31H (the opening length L31 × the opening width W31 × the number of first openings 31H) is 20% or greater and 40% or less of the area of the outermost surface 30S (the length L30 × the width W30) of the outer member 30. The unit region SU in the outermost surface 30S of the outer member 30 may be a minimal repeating unit in the outermost surface 30S, and it does not have to have an area, for example, including four first openings 31H but may also be a region including, for example, a single first opening 31H and its surrounding area. The occupancy rate of the first openings 31H in the unit area of the outermost surface 30S is 40% or less. This prevents the formation of recesses in the connective tissue at portions formed on the inside surface 20S of the inner member 20 opposing the first openings 31H. In addition, since the occupancy rate of the first openings 31H in the unit area of the outermost surface 30S is 20% or greater, the necessary amount of living tissue material reaching the inside surface 20S of the inner member 20 is obtained through the movement of the living tissue material through the first openings 31H.

Preferably, the distance between adjacent first openings 31H in the extending and circumference directions of the outer member 30 is 2.0 mm or greater and 5.0 mm or less. When the distance between adjacent first openings 31H is 2.0 mm or greater, the first openings 31H each function as an individual opening, from the viewpoint of the living tissue material, and living tissue material can evenly enter each of the first openings 31H. When the distance between adjacent first openings 31H is 5.0 mm or less, this limits differences between the amount of living tissue material entering the first openings 31H.

A method for forming connective tissue using the tissue body formation device 10 will now be described.

Living tissue material is present in an environment of a living body that includes, for example, mammals such as a human, a dog, a cow, a pig, a goat, and a sheep; birds; fish; and other animals. The environment is subcutaneous in the limbs, shoulder, back, abdomen, and the like, and the abdominal cavity. In addition, living tissue material is present in an environment such as an artificial environment containing the living tissue material. When a tissue body formation device 10 is implanted in a living body, the living body undergoes a minimal incision under sufficient anesthesia. Then, the incision is sutured following the implantation of the tissue body formation device 10.

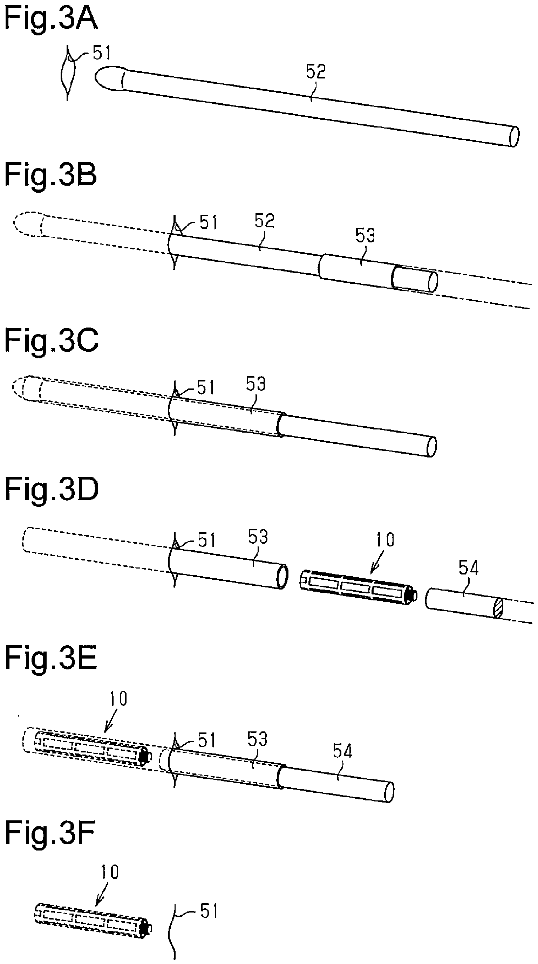

For example, as illustrated in Fig. 3A, when the tissue body formation device 10 is implanted in a living body, an insertion inlet 51 is formed first, by incision, in the surface of the living body. Then, a guide bar 52 having a round distal end is inserted into the living body through the insertion inlet 51. Further, as illustrated in Figs. 3B and 3C, an insertion pipe 53 having a round tubular shape is inserted into the living body through the insertion inlet 51. The insertion pipe 53 is moved over the outer circumference of the guide bar 52 in the extending direction of the guide bar 52. Then, as illustrated in Figs. 3D and 3E, the tissue body formation device 10 is inserted into the insertion pipe 53 and is pushed toward an inside of the living body from an outside of the living body by a push rod 54. Next, as illustrated in Fig. 3F, the insertion pipe 53 is pulled out of the living body by moving the insertion pipe 53 over the outer circumference of the push rod 54. Then, the push rod 54 is pulled out of the insertion inlet 51 thereby implanting the tissue body formation device 10 in the living body.

After implantation of the tissue body formation device 10 in the living body, connective tissue is formed on the outermost surface 30S of the outer member 30, and living tissue material enters the gap between the inner member 20 and the outer member 30 through each of the first communication parts 31 and the second communication parts 43. The living tissue material entering the gap between the inner member 20 and the outer member 30 forms connective tissue that fills the gap. During this formation, passages communicating the outside of the tissue body formation device 10 with the gap between the inner member 20 and the outer member 30 are formed by the first communication parts 31 and the second communication parts 43, which can provide connective tissue in the gap between the inner member 20 and the outer member 30 within a shorter period of time.

The tissue body formation device 10 implanted in an environment where the living tissue material is present is removed from the environment after a predetermined implantation period of time in which connective tissue is formed elapses. When the tissue body formation device 10 is removed from the living body, the living body first undergoes a minimal incision under sufficient anesthesia. Then, after removal of the tissue body formation device 10, the incision is sutured.

The removal of the tissue body formation device 10 is carried out, for example, by a procedure in a reversed order with respect to the above-mentioned implantation into the living body. Specifically, a removing rod fixed to the attached portion 23 is inserted from the insertion inlet 51 and fixed to the attached portion 23. Then, a tubular blade is moved over an outer circumference of the removing rod from the outside of the living body to the inside of the living body to cut and separate connective tissue formed inside the tissue body formation device 10 from connective tissue formed outside the tissue body formation device 10. Next, the removing rod is moved inside the tubular blade in an extending direction of the removing rod and is pulled out together with the tissue body formation device 10 from the living body through the insertion inlet 51. Finally, the tubular blade is pulled out through the insertion inlet 51, completing removal of the tissue body formation device 10 out of the living body. For the tissue body formation device 10 removed from the environment where living tissue material is present, the inner member 20, the outer member 30 and the lid 40 are mechanically separated from each other and connective tissue is removed from the inside surface 20S, i.e., a tissue forming surface, of the inner member 20. Accordingly, the connective tissue filled in the gap of hollow between the inside surface 20S and the outermost surface 30S, i.e., a covering surface, of the outer member 30 can be removed from the inside surface 20S of the inner member 20 by cutting and separating connective tissue formed on the outermost surface 30S of the outer member 30 from the outermost surface 30S and by separating the inner member 20 from the outer member 30.

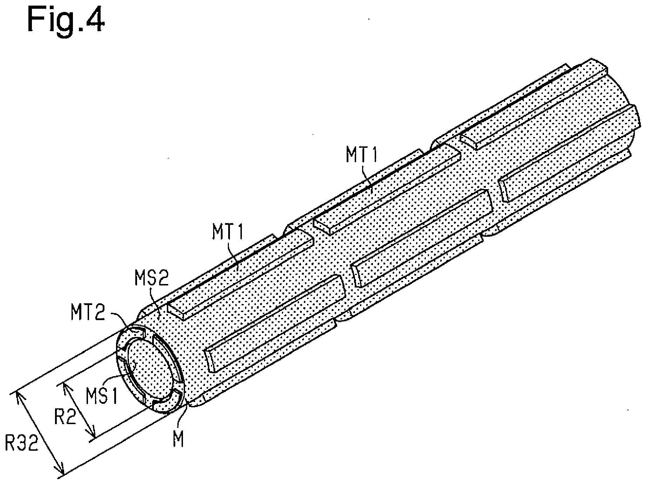

As illustrated in Fig. 4, connective tissue M formed by the use of the tissue body formation device 10 has a tubular shape corresponding to the shape of the tissue body formation device 10. The connective tissue M has an inner circumferential surface MS1 that has a shape corresponding to the inside surface 20S of the inner member 20, and has an inner diameter corresponding to the gap inner diameter R2. The connective tissue M has an outer surface MS2 that has a shape corresponding to the inside surface of the outer member 30 and an outer diameter corresponding to the gap outer diameter R32. Furthermore, the connective tissue M has an outer surface MS2 that includes projection tissue parts MT1 corresponding to the first communication parts 31, and has an end surface that includes projection tissue parts MT2 corresponding to the second communication parts 43. Accordingly, the use of the tissue body formation device 10 obtains connective tissue M with structurally improved precision.

When connective tissue formed by the tissue body formation device 10 is used for xenograft, it is preferred that immunogen removal treatments be performed such as decellularization treatment, dehydration treatment and fixing treatment to prevent or suppress rejection after implantation. The decellularization treatment includes, for example, ultrasonic treatment, surfactant treatment, and a process of washing to elute the extracellular matrix by enzymatic treatment such as collagenase. In the dehydration treatment, connective tissue M is washed by water-soluble organic solvent such as methanol, ethanol, and isopropyl alcohol. In the fixing treatment, connective tissue M is soaked into aldehyde compounds such as glutaraldehyde and formaldehyde.

Dimensions of the above-mentioned tissue body formation device 10 will now be described.

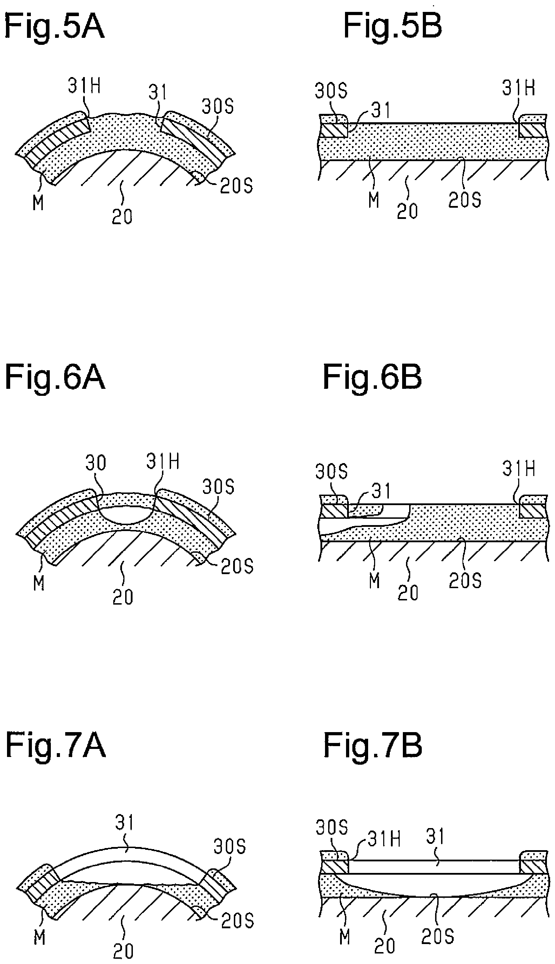

As illustrated in Fig. 5A, in the tissue body formation device 10 implanted in an environment where living tissue is present, some of the living tissue material enters the gap between the inner member 20 and the outer member 30 through the first communication parts 31 and fills the gap, forming connective tissue M. Here, as illustrated in Fig. 5B, part of the connective tissue M corresponding to the first communication part 31 has a projection tissue MT1 formed so as to fill the first opening 31H.

Now, when the occupancy rate of the first openings 31H per unit area of the outermost surface 30S is less than 20%, and particularly when the opening width W31 of the first opening 31H is less than 0.5 mm, as illustrated in Fig. 6A, part of the connective tissue M facing the first communication part 31 tends to have a recess that is not filled with the connective tissue M. As illustrated in Fig. 6B, the recess in the connective tissue M is formed because living tissue material entering in the first opening 31H clogs part of the first opening 31H before it reaches the inside surface 20S of the inner member 20. The recess is formed so as to face part of the first opening 31H in an extending direction of the first opening 31H. The recess formed in the connective tissue M produces large differences in the thickness of the connective tissue M and lowers the structural precision of the connective tissue M.

In addition, also when the first communication part 31 has a depth of greater than 2.0 mm, living tissue material entering the first opening 31H will likely remain in the first communication part 31. Furthermore, also when (the gap outer diameter R32 - the gap inner diameter R2) / 2 is less than 0.5 mm, living tissue material entering the gap between the inner member 20 and the outer member 30 will likely clog the gap before it extends over then entire inside surface 20S of the inner member 20. This will also likely produce differences in the thickness of the connective tissue M.

When the occupancy rate of the first openings 31H per unit area of the outermost surface 30S is 40% or greater, and particularly when (the gap outer diameter R32 - the gap inner diameter R2) / 2 is greater than 5.0 mm, as illustrated in Fig. 7A, part of the connective tissue M corresponding to the first communication part 31 will be provided with a large recess that is not filled with the connective tissue M. As illustrated in Fig. 7B, the recess is formed in the connective tissue M because of insufficient ingress of living tissue material into the first opening 31H. The recess is formed in an entire area of the first opening 31H in the extending and width directions of the first opening 31H. This recess also causes large differences in the thickness of the connective tissue M and lowers the structural precision of the connective tissue M.

In this point of view, with the tissue body formation device 10, the occupancy rate of the first openings 31H per unit area of the outermost surface 30S is 20% or greater and 40% or less, which prevents formation of recesses in the connective tissue, formed on the inside surface 20S of the inner member 20, at the locations opposing the first openings 31H.

### [Test example]

A test example for members or parts of a tissue body formation device 10 will now be described.

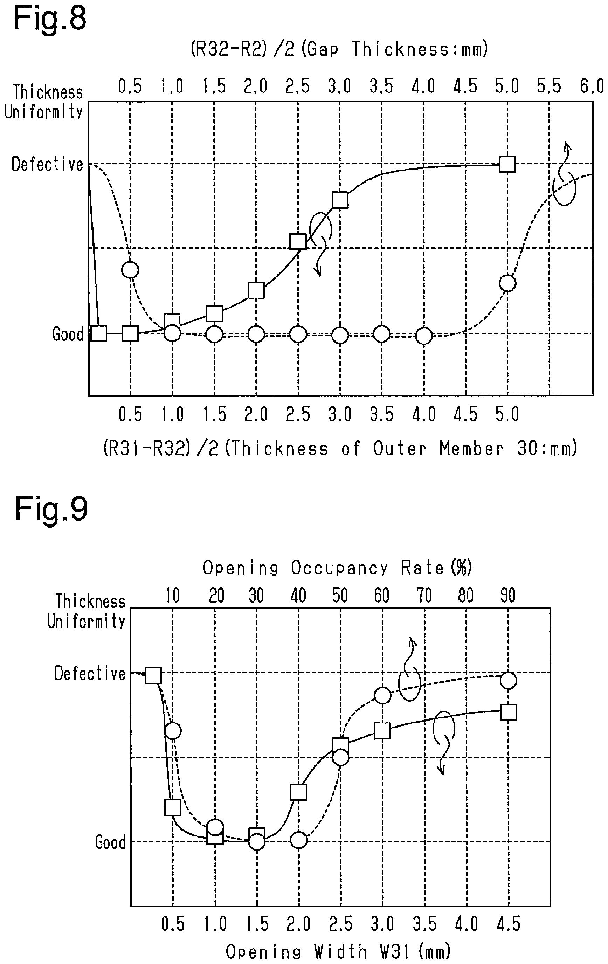

Each of dimensions (a) to (e) of the tissue body formation device 10 is changed from the reference dimensions listed below to determine structural precision of connective tissue M. The results of evaluation for the structural precision of the connective tissue M is shown in Figs. 8 and 9.

### [Reference dimension]

(Outermost diameter R31 - gap outer diameter R32) / 2 : 2.0 mm
(Gap outer diameter R32 - gap inner diameter R2) / 2 : 2.0 mm
Distance between first openings 31H in a circumference direction : 2.0 mm
Opening width W31 : 0.5 mm
Occupancy rate of first openings 31H : 20%
Implantation period of tissue body formation device 10: one month
   (a) (Outermost diameter R31 - gap outer diameter R32) / 2 : between 0.1 mm and 4.5 mm
   (b) (Gap outer diameter R32 - gap inner diameter R2) / 2 : between 0.1 mm and 9.0 mm
   (c) Distance between first openings 31H in a circumference direction : between 1.0 mm and 15.0 mm
   (d) Opening width W31 : between 0.5 mm and 5.0 mm
   (e) Occupancy rate of first opening 31H : between 10% and 90%

As illustrated in Fig. 8, at a level where (the outermost diameter R31 - the gap outer diameter R32) / 2 was between 0.1 mm and 2.0 mm, the connective tissue M was determined to have a thickness that corresponds to a dimension of (the gap outer diameter R32 - the gap inner diameter R2) / 2 and was determined to have sufficient uniformity in the extending and circumference directions of the connective tissue M. In particular, at a level where (the outermost diameter R31 - the gap outer diameter R32) / 2 was 0.5 mm or below, the connective tissue M was determined to have superior uniformity. At a level where (the outermost diameter R31 - the gap outer diameter R32) / 2 was greater than 2.0 mm, the uniformity in the thickness of the connective tissue M was determined to translate into declination along with the increment of the difference until (the outermost diameter R31 - the gap outer diameter R32) / 2 became 3.0 mm. In addition, at a level where (the outermost diameter R31 - the gap outer diameter R32) / 2 was greater than 3.0 mm, as a result of an excessive thickness of the outer member 30, i.e., as a result of an excessive depth of the first communication part 31, the connective tissue M was determined to have a part where no connective tissue M was formed in the gap between the inner member 20 and the outer member 30.

In addition, at a level where (the gap outer diameter R32 - the gap inner diameter R2) / 2 was between 0.5 mm and 5.0 mm, the connective tissue M was determined to have a thickness of dimension corresponding to (the gap outer diameter R32 - the gap inner diameter R2) / 2 and was determined to have sufficient uniformity in the extending and circumference directions of the connective tissue M. In contrast, at a level where (the gap outer diameter R32 - the gap inner diameter R2) / 2 was less than 0.5 mm, the connective tissue M was determined to have a thickness with significantly deteriorated uniformity such that it was determined to have a part where no connective tissue M was formed in the gap between the inner member 20 and the outer member 30. Furthermore, at a level where (the gap outer diameter R32 - the gap inner diameter R2) / 2 was greater than 5.0 mm, the connective tissue M was determined to have a part around the first communication part 31 where no connective tissue M was filled. In addition, in a larger outcome of (the gap outer diameter R32 - the gap inner diameter R2) / 2, the connective tissue M was determined to have greater recesses at locations opposing the first openings 31H.

Moreover, at a level where the distance between adjacent first openings 31H is 2.0 mm or greater and 5.0 mm or less, the connective tissue M was determined to have a thickness corresponding to (the gap outer diameter R32 - the gap inner diameter R2) / 2 and was determine to have a thickness with sufficient uniformity. In contrast, at a level where the distance between adjacent first openings 31H in the circumference direction is less than 2.0 mm or greater than 5.0 mm, the connective tissue M had a small thickness, and in order for the connective tissue M to have a sufficient thickness, an implantation period exceeding one month was needed.

As illustrated in Fig. 9, also at a level where the opening width W31 was between 0.5 mm and 2.0 mm, the connective tissue M was determined to have a thickness corresponding to (the gap outer diameter R32 - the gap inner diameter R2) / 2 and was determined to have sufficient uniformity in the extending and circumference directions of the connective tissue M. In contrast, at a level where the opening width W31 is less than 0.5 mm, the connective tissue M was determined to have a void part opposing the first opening 31H where no connective tissue M was formed. In addition, at a level where the opening width W31 was greater than 2.0 mm, the connective tissue M was determined to have recesses at locations opposing the first openings 31H. Furthermore, it was determined that the larger the opening width W31, the greater the recesses formed at locations opposing the first openings 31H.

In addition, also at a level where the occupancy rate of the first openings 31H was 20% or greater and 40% or less, the connective tissue M was determined to have a thickness corresponding to (the gap outer diameter R32 - the gap inner diameter R2) / 2 and was determined to have a thickness with sufficient uniformity in the extending and circumference directions of the connective tissue M. In particular, at a level where the occupancy rate of the first openings 31H was between 30% and 40%, the connective tissue M was determined to have a thickness with further sufficient uniformity. In contrast, at a level where the occupancy rate of the first openings 31H was less than 20%, the connective tissue M was determined to have a larger number of thin portions that were locally formed in an area surrounded by the outer member 30. Furthermore, at a level where the occupancy rate of the first openings 31H was greater than 40%, the connective tissue M was determined to have recesses at locations opposing the first openings 31H.

The first embodiment described has the following advantages.
(1) The opening width W31 is 0.5 mm or greater. This arrangement prevents clogging of the first openings 31H caused by living tissue material entering the first openings 31H and consequently prevents clogging of the first openings 31H during formation of the connective tissue M. Thus, structural precision of the connective tissue M is not lowered.
(2) The occupancy rate of the first openings 31H per unit area of the outermost surface 30S is 20% or greater. This obtains the necessary amount of living tissue material reaching the inside surface 20S as the living tissue material moves.
(3) The occupancy rate of the first openings 31H per unit area of the outermost surface 30S is 40% or less, which prevents formation of recesses in the connective tissue M at locations opposing the first openings 31H.
(4) The distance between the inner member 20 and the outer member 30 is 0.5 mm or greater, which prevents clogging of a gap between the inner member 20 and the outer member 30 by the connective tissue M before the connective tissue M extends over the entire inside surface 20S.
(5) The distance between the inner member 20 and the outer member 30 is 5.0 mm or less. This prevents formation of a portion in the gap between the inner member 20 and the outer member 30 that is not filled with the connective tissue M and prevents formation of recesses at locations opposing the first openings 31H.
(6) The first communication part 31 has a depth of 2.0 mm or less. This prevents clogging of the first communication parts 31 before the living tissue material entering the first communication part 31 extends over the entire inside surface 20S.
(7) The distance between adjacent first openings 31H in the circumference direction is 2.0 mm or greater. This allows each first opening 31H to act as a separate opening in relation with the living tissue material and this prevents differences in the amount of the living tissue material entering each first opening 31H.
(8) The distance between adjacent first openings 31H in the circumference direction is 5.0 mm or less. This prevents unneeded biasing or distortion in portions of the inside surface 20S of the inner member 20 covered by the outer member 30. Further, the formation of portions that are not filled with the connective tissue M is limited in the gap between the inner member 20 and the outer member 30.

### SECOND EMBODIMENT

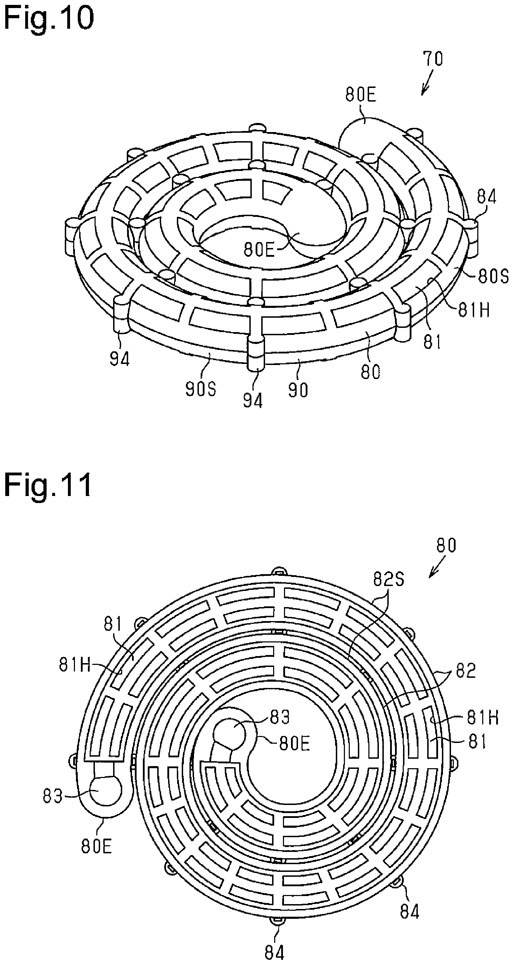

A second embodiment of the tissue body formation device will now be described with reference to Figs. 10 to 13. The tissue body formation device of the second embodiment has a contour differing from that of the first embodiment. The description below mainly focuses on differences from the first embodiment.

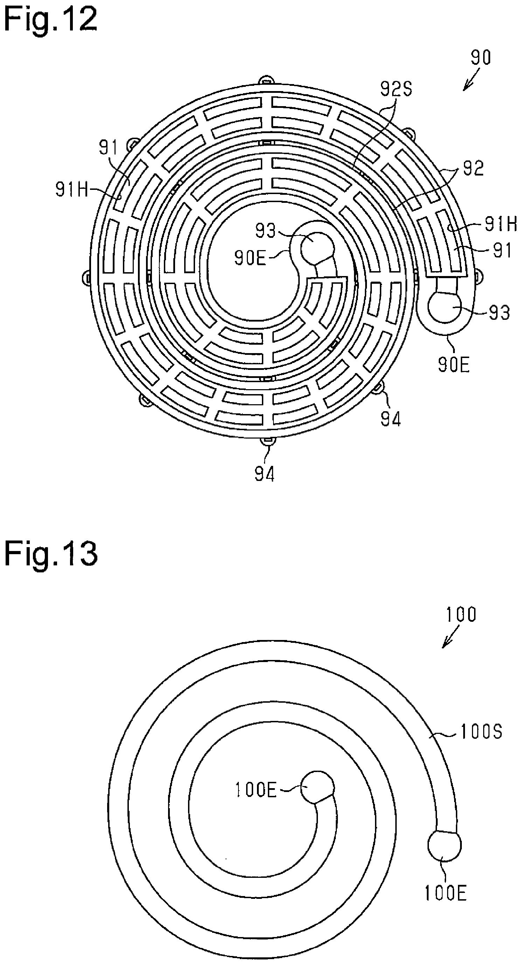

Referring to Fig. 10, a tissue body formation device 70 is a device that is embedded or implanted in an environment where living tissue material is present to form connective tissue. The tissue body formation device 70 includes an upper outer member 80 and a lower outer member 90 that form a covering member. The upper outer member 80 and the lower outer member 90 together provide a single tubular portion shaped to include two or more rings as an example of the covering member. The single tubular portion formed by the upper and lower outer members 80, 90 has an interior in which an inner ring-shaped member 100 (see Fig. 13), which is an example of the tubular portion, having two or more rings is arranged. The inner ring-shaped member 100 is ring-shape in conformance with the ring shape of the tubular portion.

The upper outer member 80 is made of semi-cylindrical acryl resin and shaped to include two or more rings, for example. The upper outer member 80 has, at its outer surface, an upper outermost surface 80S that is part of the outer surface of the tissue body formation device 70 and is the surface forming the covering surface. The upper outermost surface 80S of the upper member 80 has a semi-cylindrical surface shape and surrounds a surface of the inner ring-shaped member 100 that is used to form connective tissue, i.e., surrounds an annular inside surface 100S that is an example of tissue forming surface. The upper outer member 80 includes a plurality of upper connecting portions 84 arranged at intervals in the extending direction of the upper outer member 80. Each of the upper connecting portions 84 projects from the upper outermost surface 80S in radially inward and outward directions of the tissue body formation device 70.

The lower outer member 90 is also made of semi-cylindrical acryl resin and shaped to include two or more rings, for example. The lower outer member 90 has, at its outermost surface, a lower outermost surface 90S that is also part of the outermost surface of the tissue body formation device 70 and defines part of the covering surface. The lower outermost surface 90S of the lower outer member 90 also has a semi-cylindrical surface shape and covers the annular inside surface 100S of the inner ring-shaped member 100. The lower outer member 90 includes a plurality of lower connecting portions 94 arranged at intervals in the extending direction of the lower outer member 90. Each of the lower connecting portions 94 projects from the lower outermost surface 90S in radially inward and outward directions of the tissue body formation device 70. The lower connecting portions 94 are coupled with the corresponding upper connecting portions 84. This fixes the upper outer member 80 and the lower outer member 90 to each other.

As illustrated in Fig. 11, the upper outer member 80 has a semi-cylindrical inside surface extending in the extending direction of the upper outer member 80. The upper outer member 80 has a plurality of upper communication parts 81 through which an outer side of the upper outer member 80 communicates with the annular inside surface 100S of the inner ring-shaped member 100. Each of the upper communication part 81 has an upper opening 81H in the upper outermost surface 80S. Each upper opening 81H has a rectangular shape extending in the extending direction of the upper outer member 80. The upper outer member 80 has two ends 80E in the extending direction of the upper outer member 80 respectively defining upper support fittings 83 recessed into a hemispherical shape to support the inner ring-shaped member 100 as an example of a supporting part.

The dimension of the upper opening 81H satisfies the conditions of the first opening 31H in the first embodiment. Specifically, the opening width of the upper opening 81H along the circumference direction of the upper outer member 80, i.e., the minimum dimension of the upper opening 81H along the direction of the upper outermost surface 80S is 0.5 mm or greater. The occupancy rate of the upper openings 81H per unit area of the upper outermost surface 80S is 20% or greater and 40% or less. Preferably the opening width of the upper opening 81H is 2.0 mm or less in order to improve structural precision of the connective tissue. In addition, it is preferable that the upper communication part 81 have a depth of 2.0 mm or less.

The upper outer member 80 shaped to have two or more rings forms a structure including a plurality of annular elements 82 continuous in the radial direction and spaced apart from each other. A large diameter annular element 82 located at the circumferentially outermost position and a small diameter annular element 82 located inside the large diameter annular element 82 are radially spaced apart from each other by 0.5 mm or greater. A distance of 0.5 mm or greater between the adjacent annular elements 82 allows easy entry of living tissue material into the upper openings 81H of the outermost surface 82S of each annular element 82 and prevents clogging of the gap caused by living tissue material entering the gap.

As illustrated in Fig. 12, the lower outer member 90 includes a plurality of lower communication parts 91 through which an outer side of the lower outer member 90 communicates with the annular inside surface 100S of the inner ring-shaped member 100. Each of the lower communication part 91 has a lower opening 91H in the lower outermost surface 90S. The lower outer member 90 includes two ends 90E in the extending direction of the lower outer member 90 respectively defining lower support fittings 93, exemplifying support parts, for supporting the inner ring-shaped member 100.

The dimension of the lower opening 91H satisfies the conditions of the first opening 31H in the first embodiment. Specifically, the opening width of the lower opening 91H in the circumference direction of the lower outer member 90, i.e., the minimum dimension of the lower opening 91H in the direction along the lower outermost surface 90S is 0.5 mm or greater. The occupancy rate of the lower openings 91H per unit area of the lower outermost surface 90S is 20% or greater and 40% or less. Preferably, the opening width of the lower opening 91H is 2.0 mm or less in order to improve structural precision of the connective tissue. In addition, it is preferable that the lower communication part 91 have a depth of 2.0 mm or less.

The lower outer member 90 shaped to include two or more rings forms a structure including a plurality of annular elements 92 continuous in the radial direction and spaced apart from each other. A large diameter annular element 92 located at a circumferentially outermost position and a small diameter annular element 92 located inside the large diameter annular element 92 are radially spaced apart from each other by 0.5 mm or greater. A distance of 0.5 mm or greater between adjacent annular elements 92 allows easy entry of living tissue material into the lower opening 91H of the outermost surface 92S of each annular element 92 and prevents clogging of the gap caused by living tissue material entering the gap.

Referring to Fig. 13, the inner ring-shaped member 100 is cylindrical and is shaped to include two or more rings. The inner ring-shaped member 100 includes two ends in the extending direction of the inner ring-shaped member 100 respectively defining spherical supported parts 100E. The distance between the annular inside surface 100S of the inner ring-shaped member 100 and the inside surface of the upper outer member 80 is set by fitting the supported parts 100E to the upper supporting fittings 83. Similarly, the distance between the annular inside surface 100S of the inner ring-shaped member 100 and the inside surface of the lower outer member 90 is set by fitting the supported parts 100E to the lower supporting fittings 93. The distance between the annular inside surface 100S and the upper outer member 80 and the distance between the annular inside surface 100S and the lower outer member 90 are substantially equal and correspond to the distance between the tissue forming surface and the outer member 30 in the first embodiment, which is preferably between 0.5 mm and 5.0 mm.

Accordingly, the second embodiment has the advantageous described below in addition to advantages (1) to (8).
(9) When connective tissue having a tubular shape is formed in a limited environment that is isotropic in two dimensional directions, it is possible to increase the length of the connective tissue in the extending direction of the connective tissue.
(10) When tubular connective tissue having a desired length is formed, it is possible to have an environment, in which the tissue body formation device 70 is implanted, that is isotropic in the two dimensional directions. For example, when the insertion inlet 51 is formed in a living body, the length of the insertion inlet 51 can be shortened thereby reducing the burden on the living body.
(11) Adjacent annular elements are spaced apart by a distance of 0.5 mm or greater so that differences between the annular elements are limited in the amount of living tissue material entering each upper opening 81H and lower opening 91H.

Each of the above-mentioned embodiments can be modified and implemented as follows.

### [Covering member]

The tissue body formation device 10 is not limited to a double tubular structure including the inner member 20 and the outer member 30 and may also have, for example, a double layered structure including a plate-like covering member having a covering surface and a plate member having a tissue forming surface covered by the covering surface. Connective tissue of a sheet-like shape is formed in a gap between the covering member and the tissue forming surface of the plate member. With such a construction, the tissue body formation device can form connective tissue M that functions as an artificial valve.

In each of the tissue body formation devices 10, 70, the distance between the covering member and the tissue forming surface does not have to be constant throughout the tissue forming surface and may be larger or smaller at locations opposing the tissue forming surface compared to other locations. In addition, the distance between the tissue forming surface and the covering member may be gradually increased or decreased in a certain direction.

The covering member is not limited to a single tubular structure such as the outer member 30 and may be a structure including, for example, two or more tubes. In addition, the covering member is not limited to a single layered plate member described above and may be a plate member having two or more layers, for example. In short, the covering member should have a structure including a covering surface partially surrounding the tissue forming surface and including a plurality of communication parts through which an outer side of the covering member communicates with the tissue forming surface.

In the tissue body formation device 70, each of the upper outer member 80 and the lower outer member 90 do not have to be shaped to include a multiple rings and may be shaped to include a curve. For example, the shape may be a combination of a straight line and a curved line as well as any one of an undulated, zigzagged, or spiral shape. A tissue body formation device including such a structure obtains advantages (9) to (11)

### [Openings]

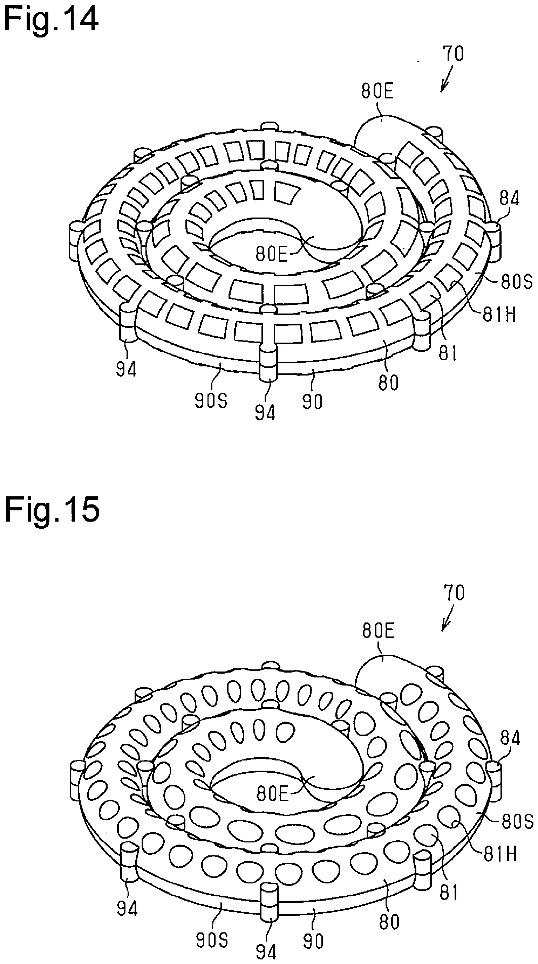

The first opening 31H, the upper opening 81H and the lower opening 91H are not limited to the rectangular shape extending in the extending direction of the covering surface and may have a rectangular shape extending in the circumference direction and a rectangular shape extending in the direction intersecting the extending direction and the circumference direction. In addition, each of the upper openings 81H and the lower openings 91H may have a square shape as illustrated in Fig. 14 or a rectangular shape with round corners as illustrated in Fig. 15, as well as a circular or oval shape and further a polygonal shape other than those. In addition, the shape may be a combination selected from a group including these shapes and the rectangular shape.

The first openings 31H do not have to be located at regular intervals in the extending and circumference directions of the outer member 30 and may be located at cyclic positions on a spiral extending in the extending direction of the outer member 30. In addition, the first openings 31H may be divided into multiple groups each including a number of first openings 31H, with the groups are sequentially positioned one by one in a certain direction, instead of the structure where the first openings 31H are sequentially positioned one by one in a certain direction.

In is only required that each of the first openings 31H have a length of 0.5 mm or greater in a certain direction on the covering surface, and the occupancy rate of the first openings 31H per unit area of the covering surface be 20% or greater and 40% or less. Preferably, for the first openings 31H described above, each first opening 31H has a minimal dimension of 2.0 mm or less. In addition, the distance between adjacent first openings 31H is preferably 2.0 mm or greater and 5.0 mm or less from the point of view of increased structural precision of the connective tissue M.

In the second embodiment, the occupancy rate of the openings per unit area of the covering surface may be less than 20% and greater than 40%. In addition, each opening may have a minimal dimension of less than 0.5 mm in the direction along the covering surface. Such a structure also obtains advantages (9) to (11). Technical concepts obtained from the modified examples are listed below.

[Appendix 1] A tissue body formation device for forming connective tissue in an environment where living tissue material is present, the device including:
a covering member having a covering surface that is a surface defining an outer surface of the tissue body formation device, wherein the covering surface covers part of a tissue forming surface that is a surface for forming the connective tissue;
wherein the covering member is a curved tubular portion and includes a plurality of communication parts through which an outer side of the tissue body formation device is in communication with the tissue forming surface, and the communication parts each having an opening in the covering surface that defines an outer surface of the tubular portion.

[Appendix 2]
The covering member includes a tubular portion shaped to have multiple rings.

[Appendix 3] The tissue body formation device according to appendix 2, wherein the tissue forming surface is a surface of an annular portion shaped to include a multiple rings in conformance with the shape of the tubular portion that is located inside the tubular portion.

[Appendix 4] The tissue body formation device according to appendix 3, wherein the tubular portion includes, at an end portion in an extending direction of the tubular portion, a supporting part for supporting the tissue forming surface so that a center of the annular portion as viewed from the extending direction coincides with a center of the tubular portion as viewed from the extending direction.

[Appendix 5] The tissue body formation device according to any one of appendixes 1 to 4, wherein the covering member includes the multiple rings in which a plurality of annular elements are arranged in the radial direction and spaced apart from each other by a distance of 0.5 mm or greater.

The end portions 80E, 90E in the second embodiment each may have a second communication part like the second communication part 43 in the first embodiment. In addition, in this modified example and in the first embodiment, the occupancy rate of the openings per unit area of the covering surface may be less than 20% and greater than 40%. Furthermore, each of the openings in the direction along the covering surface may have a minimal dimension of less than 0.5 mm. Even with such a configuration, the connective tissue M obtains a thickness. Further, uniform thickness is obtained at locations opposing the end portions 30E, 80E, 90E. A technical concept obtained from this modified example will be appended below.

[Appendix 6] A tissue body formation device for forming connective tissue in an environment where living tissue material is present, the device including:
a covering member having a covering surface that defines an outer surface of the tissue body formation device, wherein the covering surface covers part of a tissue forming surface that defines a surface for forming the connective tissue;
wherein the covering member has a tubular shape extending in one direction and a communication part through which an outer side and an inner side of the covering member are in communication at an end surface in an extending direction of the covering member.

It would be obvious for a person skilled in the art that the present invention may be embodied in other particular forms without departing from the technical ideas of the invention. For example, one or more of the members or elements described in the embodiments (or one or more of the aspects of the embodiments) may be excluded or combined together. The scope of the present invention should be determined over full scope of equivalents to which the claims are entitled with reference to the appended claims.

### DESCRIPTION OF REFERENCE CHARACTERS

M) connective tissue; R2) gap inner diameter, SU) unit area; L30) length; L31) opening length; MS1) inner circumferential surface; MS2) outer surface; MT1) projected tissue; MT2) projected tissue; R31) outermost diameter; R32) gap outer diameter; W30) width; W31) opening width (minimal dimension); 10, 70) tissue body formation device; 20) inner member; 20S) inside surface (tissue forming surface); 21) shaft member; 22) inner tubular member; 23) attached portion; 30) outer member (covering member); 30E, 80E, 90E) end portion, 30S: outermost surface (covering surface), 31: first communication part; 31H) first opening; 32) fitted portion; 40) lid; 40S) circumference surface; 41) fitting claw; 42) support hole; 43) second communication part; 43H) second opening; 51) insertion inlet; 52) guide bar; 53) insertion pipe; 54) push rod; 80) upper outer member; 80S) upper outermost surface; 81) upper communication part; 81H) upper opening; 83) upper support fitting; 90) lower outer member; 90S) lower outermost surface; 91) lower communication part; 91H) lower opening; 93) lower support fitting; 100) inner ring-shaped member; 100E) supported part; 100S) annular inside surface

## Claims

1. A tissue body formation device for forming connective tissue in an environment where living tissue material is present, the device comprising:
a covering member having a covering surface that is a surface defining an outer surface of the tissue body formation device, wherein the covering surface covers part of a tissue forming surface that is a surface for forming the connective tissue;
wherein the covering member includes a plurality of communication parts through which an outer side of the tissue body formation device is in communication with the tissue forming surface, and the communication parts each have an opening in the covering surface;
the openings each have a minimal dimension of 0.5 mm or greater in a direction extending along the covering surface; and
the openings have an occupancy rate of 20% or greater and 40% or less per unit area of the covering surface.

2. A tissue body formation device for forming connective tissue in an environment where living tissue material is present, the device comprising:
an inner member having a tissue forming surface that is a surface for forming the connective tissue; and
a covering member configured to be separable from the inner member and including a covering surface that is a surface forming an outer surface of the tissue body formation device and covers part of the tissue forming surface of the inner member, wherein a hollow gap extends between the tissue forming surface and the covering surface, and the covering member allows for removal of the connective tissue by cutting connective tissue with which the gap is filled with from other connective tissues formed on the tissue forming surface and separating the inner member and the covering member, wherein
the covering member has a plurality of communication parts through which an outer side of the covering member is in communication with the tissue forming surface, each of the communication parts including an opening in the covering surface,
the openings each have a minimal dimension of 0.5 mm or greater in a direction extending along the covering surface; and
the openings have an occupancy rate of 20% or greater and 40% or less per unit area of the covering surface, the per unit area being a structural minimum repeating unit of the covering surface.

3. The tissue body formation device according to claim 1 or 2, wherein a distance between the tissue forming surface and the covering member is 0.5 mm or greater.

4. The tissue body formation device according to claim 3 wherein a distance between the tissue forming surface and the covering member is 5.0 mm or less.

5. The tissue body formation device according to any one of claims 1 to 4, wherein the communication part has a depth of 2.0 mm or less.

6. The tissue body formation device according to any one of claims 1 to 5, wherein the distance between adjacent ones of the openings is 2.0 mm or greater and 5.0 mm or less.

7. The tissue body formation device according to any one of claims 1 to 6, wherein
the covering member includes a curved tubular part, and
the covering surface includes an outer surface of the tubular part.

8. The tissue body formation device according to any one of claims 1 to 6, wherein
the covering member includes a tubular portion shaped to have multiple rings, and
the covering surface includes an outer surface of the tubular portion.

9. The tissue body formation device according to claim 8, wherein the tissue forming surface includes a surface of an annular portion shaped to have multiple rings in conformance with the tubular portion and is located inside the tubular portion.

10. The tissue body formation device according to claim 9, wherein the tubular portion includes, at an end portion of the tubular portion in the extending direction, a supporting part for supporting the tissue forming surface so that a center of the annular portion as viewed from the extending direction coincides with a center of the tubular portion as viewed from the extending direction.

11. The tissue body formation device according to any one of claims 7 to 10, wherein the covering member is shaped to include multiple rings so that a large diameter annular element and a small diameter annular element are spaced part in the radial direction by a distance of 0.5 mm or greater.

12. The tissue body formation device according to any one of claims 1 to 11, wherein
the communication part is a first communication part, and
the covering member includes a tubular shape extending one direction and has, at an end surface of the covering member in the extending direction, a second communication part through which an outer side of the covering member is in communication with an inner side of the covering member.
